(19) Europäisches Patentamt
European Patent Office
Office européen des brevets

(11) Veröffentlichungsnummer: **0 005 803**
**B1**

(12) # EUROPÄISCHE PATENTSCHRIFT

(45) Veröffentlichungstag der Patentschrift: **13.05.81**

(51) Int. Cl.³: **C 01 B 31/00,**
C 07 C 51/58, C 07 B 9/00

(21) Anmeldenummer: **79101600.9**

(22) Anmeldetag: **24.05.79**

(54) **Verfahren zur Herstellung von Carbonyl-Difluoriden.**

(30) Priorität: **01.06.78 DE 2823981**

(43) Veröffentlichungstag der Anmeldung:
**12.12.79 Patentblatt 79/25**

(45) Bekanntmachung des Hinweises auf die Patenterteilung:
**13.05.81 Patentblatt 81/19**

(84) Benannte Vertragsstaaten:
**DE FR GB IT**

(56) Entgegenhaltungen:
**DE - A - 2 105 907**
**US - A - 2 836 622**
**US - A - 3 088 975**
**US - A - 3 253 029**

(73) Patentinhaber: **HOECHST AKTIENGESELLSCHAFT**
**Zentrale Patentabteilung Postfach 80 03 20**
**D-6230 Frankfurt/Main 80 (DE)**

(72) Erfinder: **Franz, Raimund, Dr.**
**Johann-Strauss-Strasse 36**
**D-6233 Kelkheim (Taunus) (DE)**

**EP 0 005 803 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Carbonyl-Difluoriden

Die Carbonyl-Difluoride Difluorphosgen und Oxalylfluorid sind wertvolle Ausgangsstoffe zur Herstellung fluororganischer Verbindungen, wie z.B. von halogenierten Methanen (vgl. Beilstein E IV 3, S. 21) oder von perfluorierten Ethern (US—A—3 250 807).

Es ist bekannt, Difluorphosgen und Oxalylfluorid durch Fluorierung der entsprechenden Dichloride (Phosgen und Oxalylchlorid) herzustellen. Als Fluorierungsmittel gelkangen dabei meist Alkalimetallfluoride, insbesondere NaF, zur Anwendung (J. Org. Chem. 1960, Seiten 25 und 2016 — 19; US—A—3 088 975), die entweder heterogen in einem geeigneten aprotischen Medium suspendiert oder aber — im Falle der Herstellung des Difluorphosgens — auch bei hohen Temperaturen in geschmolzener Form eingesetzt werden können.

Die Fluorierung im heterogenen System besitzt jedoch den Nachteil, daß die Korngröße und der Grad der Trockenheit des Fluorids den Reaktionsverlauf erheblich beeinflussen; Reaktionen im heterogenen System verlaufen außerdem generell langsamer als Reaktionen in homogenen Systemen, so daß für zweckmäßige Reaktionszeiten höhere Temperaturen erforderlich werden. Höhere Temperaturen sind aber im Falle thermisch labiler Substrate oder Produkte wie des Oxalylfluorids ungünstig. So entsteht bei der Herstellung von Oxalylfluorid durch Fluorierung von Oxalylchlorid mit Alkalimetallfluoriden nach dem Stand der Technik bei Temperaturen bis etwa 120°C stets Fluorphosgen als unerwünschtes Nebenprodukt.

Die Fluorierung mit geschmolzenem Alkalimetallfluorid ist wegen der thermischen Labilität des Oxalylfluorids zu dessen Herstellung nicht möglich. Diese Methode kommt nur zur Herstellung des — thermisch stabileren — Difluorphosgens in Frage. In diesem Falle sind geeignete hitze- und korrosionsbeständige Apparaturen erforderlich.

Es ist weiterhin bekannt, Carbonsäurefluoride aus den entsprechenden Carbonsäurechloriden durch Fluorierung mit Fluorwasserstoff zu gewinnen (Houben-Weyl, Methoden der Organischen chemie 4. Auflage Band V/3, G. Thieme Verlag, Stuttgart 1962, Seite 119 ff). Fluorwasserstoff ist zwar ein erheblich billigeres Fluorierungsmittel als Alkalimetallfluoride, doch für die Herstellung von Difluorphosgen aus Phosgen nur schwierig und für die Herstellung von Oxalylfluorid aus Oxalylchlorid praktisch überhaupt nicht verwendbar.

Zur Herstellung von Difluorphosgen aus Phosgen und Fluorwasserstoff muß im Eisenautoklaven in Gegenwart von Aktivkohle auf 140 bis 150°C erhitzt werden (Beilstein E III Seite 22).

Difluorphosgen kann aber auch bei atmosphärischem Druck aus Phosgen und Fluorwasserstoff — allerdings bei vorzugsweise noch höherer Temperatur (insbesondere bei 150 bis 300°C) in Gegenwart von Aktivkohle — hergestellt werden (US—A—2 836 622). Das dabei zunächst entstehende Gemisch aus Difluorphosgen und Chlorwasserstoff muß dann noch mit Hilfe von Chlorwasserstoff-bindenden Mitteln (Alkalifluoride, $SO_3$ oder $P_2O_5$) getrennt werden.

Nach der US—A—3 253 029 können u.a. niedere aliphatische Nitrile (wie z.B. Acetonitril) eine Anreicherung von Difluorphosgen in Gemischen mit Chlorwasserstoff bewirken, da die aliphatischen Nitrile Chlorwasserstoff in stärkerem Maße als Difluorphosgen binden sollen. Von einer anderen als der Anreicherungsfunktion für Difluorphosgen/Chlorwasserstoff-Gemische ist in der US—A—3 253 029 nirgends die Rede.

Die Fluorierung von Oxalylchlorid mit Fluorwasserstoff gelingt deswegen nicht, weil beim Vermischen der Reaktionspartner praktisch keine Reaktion stattfindet und bei höheren Temperaturen Zersetzung eintritt.

Es war daher wünschenswert und bestand die Aufgabe, ein besseres Verfahren zur Herstellung der Carbonyl-difluoride Difluorphosgen und Oxalylfluorid zu finden.

Diese Aufgabe konnte erfindungsgemäß durch Fluorierung von Phosgen bzw. von Oxalylchlorid mit Fluorwasserstoff in Gegenwart von Acetonitril gelöst werden.

Erfindungsgegenstand ist somit ein Verfahren zur Herstellung von Carbonyldifluoriden der Formel

$$F—(CO)_n—F$$

worin n = 1 oder 2,
durch Fluorierung der entsprechenden Carbonyldichloride, das dadurch gekennzeichnet ist, daß man die Fluorierung mit Fluorwasserstoff in Gegenwart von Acetonitril durchführt.

Wenn in der Formel n = 1, handelt es sich um Difluorphosgen, wenn n = 2 um Oxalylfluorid. Zur Herstellung des Difluorphosgens ist von Phosgen $COCl_2$, zur Herstellung des Oxalylfluorids von Oxalylchlorid $(CO)_2Cl_2$ auszugehen. Natürlich könnte man auch von den entsprechenden Bromverbindungen (Dibromophosgen bzw. Oxalylbromid) ausgehen, doch bringt dies keine Vorteile, da die Bromide kostspieliger und thermisch labiler sind als die Chloride. Die anzuwendende Fluorwasserstoffmenge soll mindestens gleich der theoretischen Menge — also mindestens 2 Mol pro Mol Carbonyldifluorid — sein; bevorzugt ist jedoch ein molarer Überschuß von etwa 50 bis 200 Mol.-%.

Für das Gelingen der Reaktion genügt schon die Gegenwart von ziemlich geringen Mengen Acetonitril; bevorzugt ist jedoch die Durchführung der Fluorierung in einer Mischung von

Fluorwasserstoff und Acetonitril, in welcher die Fluorwasserstoffkonzentration etwa 10 bis 99, vorzugsweise etwa 30 bis 40 Mol.-% beträgt. Wenn die Fluorwasserstoffkonzentration in dieser Mischung nicht höher als 50 Mol.-% ist, kann die Reaktion auch in Apparaturen aus Borsilikatglas durchgeführt werden.

Das überraschend gute Gelingen der Reaktion kommt wahrscheinlich teils durch die besonderen lösungsvermittelnden Eigenschaften, teils durch die spezielle Basizität des Acetonitrils zustande.

Als günstiger Temperaturbereich für die erfindungsgemäße Fluorierung kommt ein solcher zwischen etawa 0 und 50, vorzugsweise zwischen etwa 10 und 40°C infrage.

Nach beendeter Reaktion wird das Reaktionsprodukt zweckmäßig destillativ, gegebenenfalls unter vermindertem Druck, aufgearbeitet.

Im Falle der Fluorierung von Oxalylchlorid besteht das sich entwickelnde Gasgemisch Chlorwasserstoff und Oxalylfluorid, wovon das letztere aufgrund der Lage seines Siede-bzw. Schmelzpunktes mit Hilfe von Kühlfallen leicht herauskondensiert werden kann.

Im Falle der Fluorierung von Phosgen besteht das Produktgemisch aus Difluorphosgen und Chlorwasserstoff, die nur durch eine aufwendige Fraktionierung oder eine nachgeschaltete chemische Reaktion mit einem geeigneten säurebindenden Agens getrennt werden können. Eine bevorzugte Variante des erfindungsgemäßen Verfahrens besteht daher darin, die Fluorierung in Gegenwart einer Chlorwasserstoff-bindenden Hilfsbase durchzuführen, die weder mit dem Substrat noch mit dem Reaktionsprodukt reagiert. Bevorzugte derartige Hilfsbasen sind tert. Amine, insbesondere Triäthylamin. Die Menge der einzusetzenden Hilfsbase soll so bemessen sein, daß der gesamte entstehende Chlorwasserstoff gebunden wird. Durch Aufarbeiten des gebildeten Hydrochlorids mit Lauge kann die Hilfsbase wieder quantitativ zurückgewonnen werden.

Nach dem erfindungsgemäßen Verfahren erhält man Difluorphosgen bzw. Oxalylfluorid in Ausbeuten bis über 70% d.Th. Das Verfahren hat gegenüber den Verfahren nach dem Stand der Technik den Vorteil, daß mit einem billigen Fluorierungsmittel auf einfache Weise — wenn gewünscht, in Glasgerätenunter Bedingungen fluoriert werden kann, die der Entstehung von Neben- bzw. Zersetzungsprodukten keinen Verschub leisten. So ist etwa bei der Herstellung von Oxalylfluorid im Gegensatz zum literaturbekannten Verfahren im entstehenden Produktgemisch kein unerwünschtes Fluorphosgen nachweisbar.

Die folgenden Beispiele sollen der weiteren Erläuterung der Erfindung dienen.

Beispiel 1

In einem 2 l — Rührkolben aus Borsilikatglas legt man eine Mischung aus 120 g Fluorwasserstoff (6 Mol) und ca. 500 g Acetonitril vor und tropft unter Kühlung 202 g Triäthylamin (2 Mol) zu. Die so erhaltene Lösung wird bei Raumtemperatur langsam mit 99 g Phosgen (1 Mol) versetzt. Auf dem Rührkolben befindet sich ein Rückflußkühler, der mit Trockeneis — Kältegemisch beschickt wird. Das entstehende Difluorphosgen durchströmt diesen Kühler und wird in einer nachgeschalteten, mit flüssiger Luft gekühlten Falle kondensiert. Die Reaktion wird durch Anlegen von Vakuum und Erwärmen der Reaktionsmischung bis max. 40°C vervollständigt. In der Kühlfalle finden sich danach ca. 60 g Rohprodukt, das nach IR-Spektrum und Gaschromatogramm nur 15—20% Kohlendioxid als Verunreinigung aufweist. Die Ausbeute beträgt somit ca. 72% d.Th.

Beispiel 2

Eine Lösung von 60 g Fluorwasserstoff (3 Mol) in ca. 250 g Acetonitril, die sich in einem Einliter-Rührkolben aus Borsilikatglas mit Rückflußkühler (—78°C) befand, wurde bei 10°C mit 99 g Phosgen (1 Mol) versetzt. Man ließ bei Raumtemperatur 2 Std. nachrühren und brachte dann das Reaktionsgemisch durch Vermindern des Druckes 3 Stunden lang zum schwachen Sieden. In einer hinter den Rückflußkühler geschalteten, mit flüssiger Luft gekühlten Falle sammelten sich 60 g eines Gemisches, das nach dem IR-Spektrum Chlorwasserstoff und Difluorphosgen als Hauptkomponenten, sowie Kohlendioxid und Fluorchlorphosgen als Nebenkomponenten enthielt.

Beispiel 3

In einer Rührapparatur aus Borsilikatglas, bestehend aus Kolben, Rückflußkühler (mit Sole von —10°C) und drei hintereinandergeschalteten Kühlfallen im Trockeneisbad, wird eine Lösung von 120 g Fluorwasserstoff (6,0 Mol) in 600 ml Acetonitril vorgelegt und bei ca. 13°C 254 g Oxalylchlorid (2.0 Mol) in 1 Std. zugetropft. Während dieser Zeit, sowie während des nachfolgenden, einstündigen Nachrührens ist bereits eine geringe Gasentwicklung zu beobachten. Diese wird anschließend durch Anlegen von Vakuum verstärkt (Wasserstrahlpumpe). In Abhängigkeit von der beobachteten Gasentwicklung senkt man den Innendruck im Lauf von ca. 2 Std. auf 60—70 mbar ab und läßt dabei Temperatur der Kühlsole von —10 auf —40°C fallen. Das Ende der Reaktion erkennt man am Aufhören der Gasentwicklung. Die in den Kühlfallen befindlichen Kondensate werden vereinigt und einer Fraktionierung unterworfen. Men erhält so 130—140 g Oxalylfluorid vom Kp. —2°C (= 69 — 74% d. Th.).

**Patentansprüche**

1. Verfahren zur Herstellung von Carbonyl-Difluoriden der Formel

$$F—(CO)_n—F$$

worin n = 1 oder 2, durch Fluorierung der entsprechenden Carbonyldichloride, dadurch gekennzeichnet, daß man die Fluorierung mit Fluorwasserstoff in Gegenwart von Acetonitril bei Temperaturen von etwa 0 bis 50, vorzugsweise von etwa 10 bis 40°C durchführt.

2. Verfahren nach Anspruch 1, dadurch gekennzeichnet, daß man die Fluorierung in einer Mischung von Fluorwasserstoff und Acetonitril durchführt, in welcher die Fluorwasserstoffkonzentration etwa 10 bis 99, vorzugsweise etwa 30 bis 40 Mol.-% beträgt.

3. Verfahren nach den Ansprüchen 1 bis 2, dadurch gekennzeichnet, daß man der Fluorierung ein Chlorwasserstoffbindendes tert. Amin, vorzugsweise Triäthylamin, zusetzt.

**Claims**

1. Process for the preparation of carbonyl difluorides of the formula

$$F{-}(CO)_n{-}F$$

in which n is 1 or 2,
by fluorination of the corresponding carbonyl dichlorides, characterized in carrying out the fluorination with hydrogen fluoride in the presence of acetonitrile at temperatures of about 0—50, preferably of about 10—40°C.

2. Process as claimed in claim 1, characterized in carrying out the fluorination in a mixture of hydrogen fluoride and acetonitrile in which the concentration of hydrogen fluoride is in the range of from about 10 to 99, preferably from about 30 to 40 mol%.

3. Process as claimed in any one of claims 1 or 2, characterized in adding to the fluorination reaction a tertiary amine binding hydrogen chloride, preferably triethylamine.

**Revendications**

1. Procédé de préparation de difluorures de carbonyles répondant à la formule

$$F{-}(CO)_n{-}F$$

dans laquelle n est égal à 1 ou à 2, par fluoration des dichlorures de carbonyles correspondants, procédé caractérisé en ce qu'on effectue la fluoration au moyen du fluorure d'hydrogène en présence d'acétonitrile à des températures d'environ 0 à 50°C, de préférence d'environ 10 à 40°C.

2. Procédé selon la revendication 1, caractérisé en ce qu'on effectue la fluoration dans un mélange de fluorure d'hydrogène et d'acétonitrile dont la concentration en fluorure d'hydrogène est d'environ 10 à 99% en moles, de préférence d'environ 30 à 40 % en moles.

3. Procédé selon l'une des revendications 1 et 2, caractérisé en ce qu'on ajoute au mélange de fluoration une amine tertiaire capable de fixer le chlorure d'hydrogène, de préférence la triéthylamine.